# EUROPEAN PATENT SPECIFICATION

(11) **EP 2 480 267 B1**
(45) Date of publication and mention of the grant of the patent: **09.01.2019**
(21) Application number: 10752392.0
(22) Date of filing: 20.08.2010
(51) Int. Cl.: G04C 17/00, G01F 23/02, G12B 11/00, G04B 1/26, A61M 5/145, A61M 5/142

(54) **VISUAL INDICATOR AND FLUID DISPENSER**
OPTISCHE ANZEIGE UND FLUID-DISPENSER
AFFICHAGE VISUEL ET DISTRIBUTEUR DE FLUIDE

(30) Priority: 21.08.2009 US 235725 P; 31.05.2010 US 349897 P
(43) Date of publication of application: 01.08.2012
(73) Proprietor: Preciflex SA, 2000 Neuchâtel (CH)
(72) Inventor: Vouillamoz, Lucien, CH-8835 Feusisberg (CH)
(74) Representative: Mötteli-Mantelli, Novella
(86) International application number: PCT/IB2010/002055
(87) International publication number: WO 2011/021098

(56) References cited:
- WO-A1-82/03556
- WO-A1-95/14497
- WO-A1-2006/118479
- WO-A2-2006/065976
- DE-A1- 4 207 122
- DE-U1-202004 000 890
- GB-A- 2 077 110
- US-A- 3 923 250
- US-A1- 2004 231 116

## Description

This invention relates to indicators and in particular analog visual indicators used to dispense a measured amount of liquid.

Analog indicators have existed since time immemorial. The hour glass, for example, uses sand or fluid which, influenced by the weight of gravity, moves from one reservoir to another by passing through a small aperture therebetween. Another example of an ancient analog indicator is the "Clepsydra", as illustrated in "Horloges Anciennes" by Richard Mühe and Horand M. Vogel, French Edition, Office du Livre, Fribourg, 1978, page 9.

Referring to **FIG. 1****,** US Patent No. 3,783,598 describes an instrument 1 having a movement 2, a drive shaft 3, cams 4, pistons 5, fluid filled capillaries 6 and a relief chamber 7 used to indicate time. Automated fluid dosage devices exist. A typical insulin pump is a computerized device that looks like a pager and is usually worn on the patient's waistband or belt. The pump is programmed to deliver small, steady doses of insulin throughout the day. Additional doses are given to cover food or high blood glucose levels. The pump holds a reservoir of insulin that is attached to a system of tubing called an infusion set. Most infusion sets are started with a guide needle, then the plastic cannula (a tiny, flexible plastic tube) is left in place, taped with dressing, and the needle is removed. The cannula is usually changed every 2 or 3 days or when blood glucose levels remain above target range. However, such devices are bulky and are not always located at a place on the body that is easy to access or read.

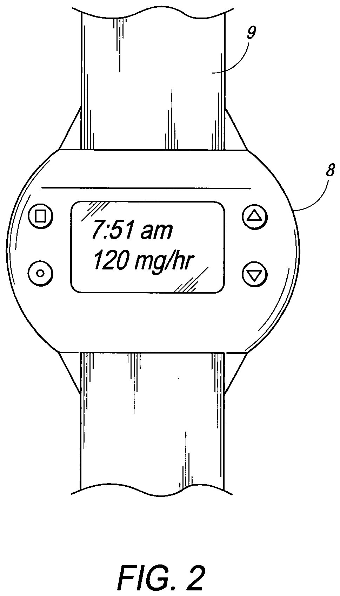

Referring to **FIG. 2****,** a wrist worn device, such as the "GLUCOWATCH" is known. This prior art device, said to be developed in 2001, has a casing 8 supported on a bracelet 9. A reservoir dispenses insulin onto a patch similar to a transdermal medication patch used for smoking cessation and hormone therapy. It therefore provides a non-invasive, needle-free method of enhancing and controlling the transport of water-soluble ionic drugs out of the skin and surrounding tissues using a low level of electrical current. DE 20 2004 000 890 U1 describes a bracelet used as a reservoir for perfume. An action on the piston allows a vaporization of the liquid.

GB 2 077 110 and WO 82/03556 disclose insulin device systems wherein the release liquid is carefully controlled.

These prior devices are cumbersome, requiring significant or dedicated space for indicating the value, lack accuracy or only are too costly for many users.

What is needed is a visual indicator that provides a quickly read indication of a measured dosage value and is inexpensive to manufacture.

### Summary of the Invention

A visual indicator display device is provided according to claim 1. Further embodiments are provided according to the claims dependent therefrom. The visual indicator includes a bracelet, a transparent capillary chamber, and a displacement member. The transparent capillary chamber is matched to an indicia and has a primary length and a width less than the primary length. The displacement member is functionally disposed at one end of the capillary chamber and is responsive to a measureable input for moving a fluid contained therein a defined amount.

An object of the invention is to provide a visual indicator which takes up minimal space.

Another object of the invention is to provide a flexible visual indicator which adapts to requirements which do not readily permit a straight, rigid indicator, such as when such indicator is worn on a wrist, ankles, a head or around or along some part of human body, or on objects such as clothes and sporting articles, .

Another object of the invention is to provide an aesthetic, comfortable, reliable and intellectually attractive indicator.

Another object of the invention is to provide a dispenser of fluids such as drugs, medication, ointment, oils or perfumes.

### Brief Description of the Drawings

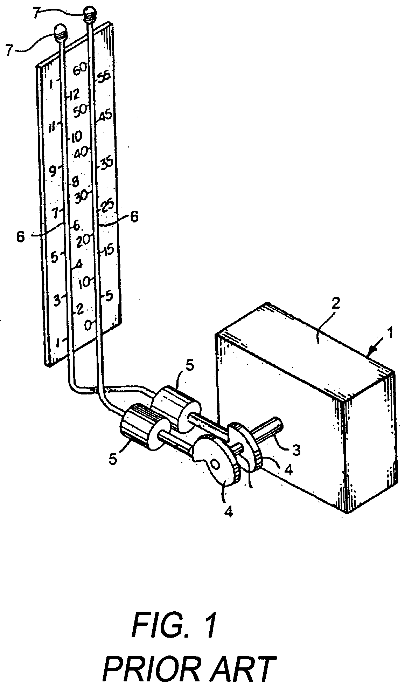

**FIG. 1** is a side, cross-sectional view of an analog indicator of the prior art.
**FIG. 2** is a top view of a second indicator of the prior art.
**FIG. 3** is a side, cross sectional view of a first embodiment of the invention.
**FIG. 4A** is a perspective view of a second embodiment of the invention.
**FIG. 4B** is a second perspective view of the second embodiment of the invention.
**FIG. 5A** is a second embodiment of the invention, used as a drug dispenser.
**FIG. 5B** is a side view of a cartridge for use in the embodiment of **FIG. 5A****.**
**FIG. 5C** is a perspective view of a cartridge for use in the embodiment of **FIG. 5A****,** shown in a flexed state.

Those skilled in the art will appreciate that elements in the figures are illustrated for simplicity and clarity and have not necessarily been drawn to scale. For example, dimensions may be exaggerated relative to other elements to help improve understanding of the invention and its embodiments. Furthermore, when the terms 'first', 'second', and the like are used herein, their use is intended for distinguishing between similar elements and not necessarily for describing a sequential or chronological order. Moreover, relative terms like 'front', 'back', 'top' and 'bottom', and the like in the description and/or in the claims are not necessarily used for describing exclusive relative position. Those skilled in the art will therefore understand that such terms may be interchangeable with other terms, and that the embodiments described herein are capable of operating in other orientations than those explicitly illustrated or otherwise described.

### Detailed Description of the Preferred Embodiment

A visual indicator display device includes a bracelet, a transparent capillary chamber, and a displacement member. The transparent capillary chamber is matched to an indicia and has a primary length and a width less than the primary length. The displacement member is functionally disposed at one end of the capillary chamber and is responsive to a measureable input for moving a fluid contained therein a defined amount.

A suitable fluid may be an oil, a lotion, or a liquid such as a drug or other medication. The displacement member is attached to one end of the capillary chamber which is responsive to a measureable input fordisplacing the indicator surface thus allowing the user to read a measurement from the indicia.

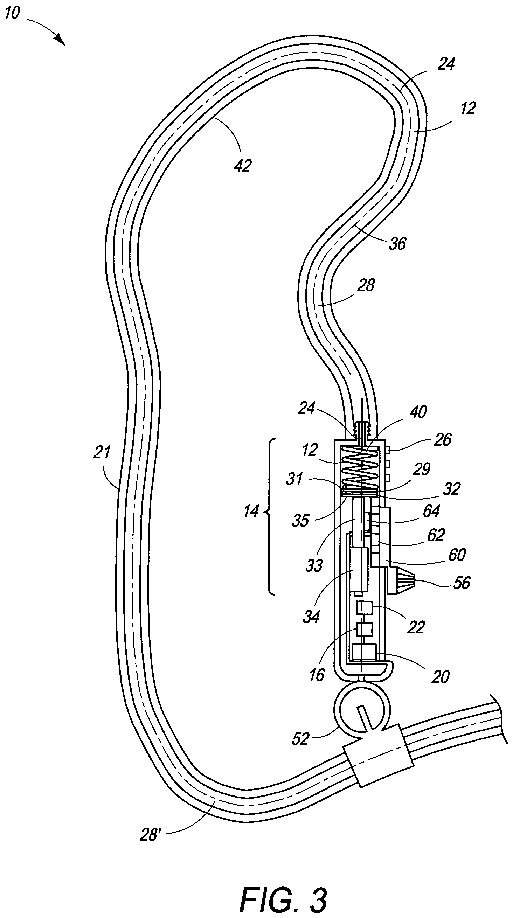

Referring to **FIG. 3****,** an analog indicator 10 of the invention indicates dosage. The indicator 10 includes a reservoir 12, a pump 14, a measuring device 16, a feedback circuit in a controller 20 and a power supply 22'. The reservoir 12 has a longitudinal axis 24 along which a indicia or a scale device 26 is disposed and is adapted for containing a fluid 28 bounded by at least an indicator surface 30. In a preferred embodiment, the pump 14 is made up of the plunger 32 mounted on a screw 33 driven by a micro motor 34. The plunger 32 generally uses an O-ring seal 29 disposed about its circumference, to seal against the fluid 28 passing between the top and bottom surface 31 and 35, respectively, of the plunger. The pump 14 pumps the fluid 28 out of the reservoir 12, and into the catheter 502. In a preferred embodiment, the measuring device 16 is an electronic clock which measures time and communicates a measured value of time to the feedback circuit 20. The feedback circuit 20, powered by the power supply 22, receives a measured time input from the measuring device 16 corresponding to a position on the scale device 26 and, in response thereto, activates the pump 14 to pump or move the fluid 28 out of the reservoir 12, until the surface 30 reaches a desired position in relation to the corresponding position on the indicia 26 (generally calibrated to equal a desired rate of dispensing of the fluid). The power supply 22 powers the pump 14 and feedback circuit 20. As shown, the reservoir 12 communicates the fluid 28 into the catheter 502. A clasp 52 connects ends of the device 10 to create a bracelet 21.

Further, optionally, an optical fiber and an LED light source illuminate the fluid 28 in the reservoir 12 in a known manner.

A potentiometer 56 regulates the voltage setting to a displacement control system 60. The displacement control system 60 includes an incremental position sensor 62, for example, the tracker NSE-5310 (the specification of which is attached as Appendix A to U.S. Provisional Application No. 61/235,725, filed 21 August 2009) located adjacent the plunger 32. This control system 60 includes encoding for direct digital output, in which a hall element array on the chip 62 is used to derive the incremental position of an external magnetic strip 64 placed adjacent the chip at a distance of approximately 0.3 mm (typically), the magnetic strip 64 being attached to the plunger 32 in order to translate therewith. This sensor array detects the ends of the magnetic strip to provide a zero reference point.

In an alternate embodiment, the power supply 22 can be solar cells, a wound watch spring, movement captured by an oscillating mass (such as used in automatic watches), or a pneumatic system storing compressed air.

To return the fluid 28 to an initial position, such as 6:00 AM, for example, the plunger 32 may be returned by a return spring 40 or a magnetic device (not shown). Other options are conceivable, of course, which include the return line 42, which allows simple reversing of the motor 34 to reset the indicator 10.

A suitable motor 34 is referred to by its trademark SQUIGGLE™, available from New Scale Technologies, Inc. of New York, USA.

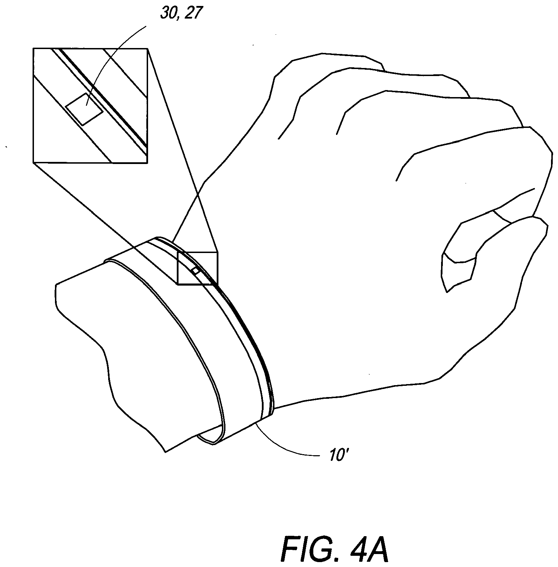

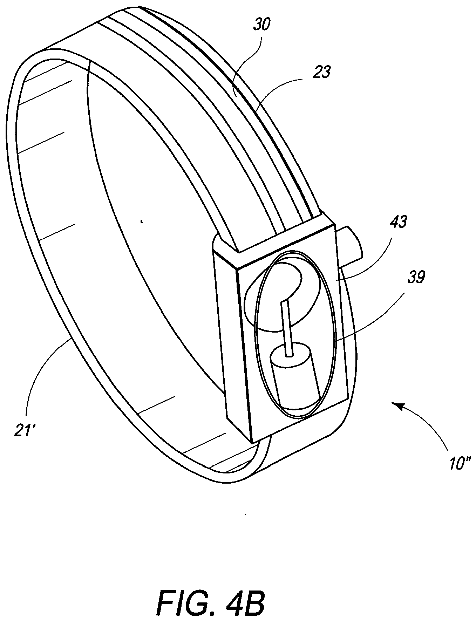

Referring now to **FIGs. 4A** and **4B****,** an application of the analog indicator of the invention is a wrist watch or necklace 10 worn around the user's wrist. The reservoir 12' may be made of a transparent or translucent material, or a mixture of transparent and translucent material, formed in any desired shape. It may be made of plastic, rubber, silicon or any suitable material. An elastic material has the advantage that the bracelet 21' may be stretched over the user's wrist. In addition, the fluidic display 23 may be supplemented with a standard watch face 39 on the casing 43.

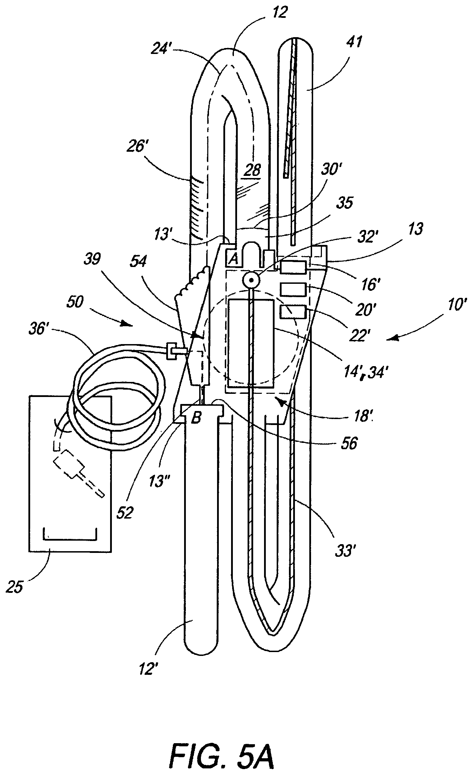

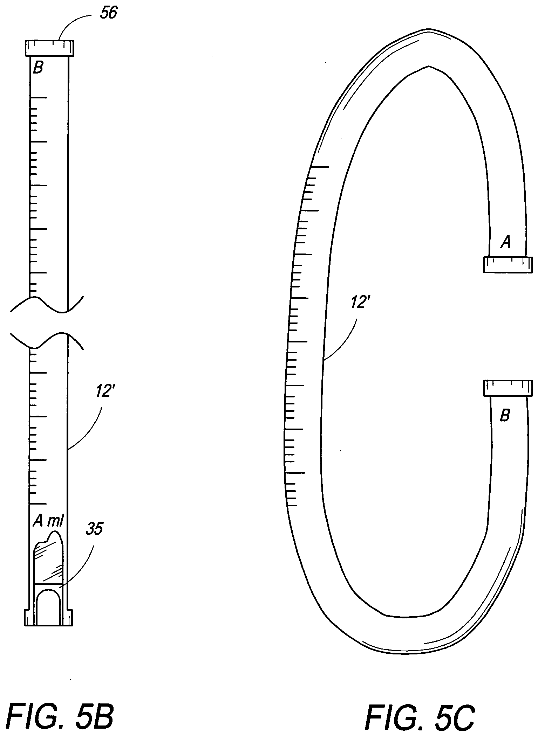

Referring now to **FIG. 5A****,** the invention may be configured as a device 10" used to administer doses of liquid drugs 28 such as insulin. In such an embodiment, the flexible tube is a disposable drug reservoir cartridge 12' attached to housing 13 containing a dosage control device 18. The device 10" is carried like a wrist watch, with the flexible cartridge 12' serving as a portion of the band thereof. The indicator 10" includes the reservoir 12', a linear drive 14', an optional feedback circuit 16', a controller 20', and a power supply 22'. The reservoir 12' has a longitudinal axis 24' along which indicia 26' is disposed and is adapted for containing the fluid 28 bounded by at least an indicator surface 30'. In a preferred embodiment, the linear drive 14' drives a spherical plunger 32' mounted on a long flexible threaded shaft 33' which is driven by a micro motor 34'. The shaft 33' is preferably made of a superelastic material such as NITINOL. The linear drive 14' drives the plunger 32' against the piston 35 (preferably made of a flexible material such as rubber) which in turn presses the fluid 28 along the reservoir 12' and ultimately through the cannula tube or catheter 36', which then guides the fluid 28 into the patient's body. The electronics of the device 10" ensures that a programmed dosage of fluid is administered at regular intervals or constantly as prescribed by a physician. Note that optionally, the fluid 28, instead of passing into a wearer's body via a cannula, charges an absorptive patch 25 worn by the patient, for slow diffusion of the drug into the patient's body through the skin. Where a medication is administered via a patch 25, the patch may include an outer layer which is semipermeable, in order to prevent the medication from evaporating before it has its intended effect (i.e. diffusion into the skin). Further, a perfume may be delivered in a similar manner. Particularly for the perfume dispensing embodiment, the patch may be located partially or entirely under the housing 13, or to the side of the housing and may be affixed thereto using a temporary adhesive rather than directly to the living organism, in order to avoid the need to attach the same to the living organism. Such a patch may be sized to be replaced in a defined area (such as circular area marked 39) against the back or any side of the housing 13, adjacent the living organism, much like a "POST-IT" note, so that replacement patches can readily replace soiled patches.

In a preferred embodiment, the number of turns of the linear drive 14' is recorded and controlled so as to ensure the proper dosage. The electronics are powered by the power supply 22'. Alternatively, the position of the piston 35 can be controlled in the manner as described in the above embodiment shown in **FIG. 3****.** The cartridge 12' installs on one side 13' of the housing 13, with its piston 35 adjacent the plunger 32', and on the other side 13", adjacent a piercing mechanism 50 which includes a piercing tube 52 connected to a slidable tab 54. The user may slide the tab 54 to cause the piercing tube 52 to pierce the upper membrane 56 of the cartridge 12', in order to permit the communication of the fluid 28 through the cannula 38 into the patient's body. Where perfume is dispensed, this piercing served to open one end of the cartridge 12' to allow the delivery of perfume into the air, or via a conductive channel (not shown), to, near, or adjacent the skin of the user (for example, directly to and through the patch).

In the embodiment using an external magnetic strip (having a magnetic characteristic where the magnetic field generated thereby increases or decreases along the length of the cartridge) attached to or integrated on the cartridge 12', the computer controller can use this to regulate the dosage administered to the patient.

As with the prior embodiment, the power supply 22' can be a battery, solar power, a wound watch spring, an oscillating mass (such as used in automatic watches), or a pneumatic system storing compressed air,

After a cartridge 12' is fully dispensed, a button (not shown) on the housing 13 can be activated to retract the plunger 32'. The piston 35 remains stationary to prevent any aspiration of fluid from the patient, should the cannula still be connected to the body. Once retracted, the device 10" can be reloaded with a replacement cartridge 12'.

As with the earlier embodiment, a suitable motor 34 is the SQUIGGLE™ motor already described.

Note, that the housing 13 can be fitted with a watch face 39 and corresponding movement (not shown), in order that the drug administration device can also serve as a wrist watch.

Optionally, the threaded rod 33' of the drug administration device 10" is enclosed in a tube 41 which connects on the side 13" of the housing 13' and wraps around the wearer's wrist to reconnect to the side 13' of the housing, giving the visual effect of a two or multi-banded wrist watch.

It is foreseen that the cartridge 12' used in such drug administration device 10" would include a chemical litmus-type indicator which would indicate whether the insulin or other drug is suitable for continued injection. This indication could be expressed by an element of the cartridge 12' changing color, from a color that indicates the fluid is suitable for use, to another color that indicates the fluid is no longer suitable for use.

Still further, the device 10" can be used as a perfume dispenser by replacing the cannula with an aspirating head which can be manually (via a dispenser head or button) or automatically (via the dosage control of the invention) operated.

It should be noted that the invention 10, 10', 10" may be made exclusive of all electronics (such as would typically be the case where the invention is positioned in the luxury watch market). In such embodiment, the power source 22" may be movement from an oscillating mass, which winds a watch spring, which powers a gear train, for which the rate of rotation is controlled by a pendulum-like regulator or oscillating disk (e.g., a balancier/turbion), which has a characteristic period, as known in the art.

Other embodiments are shown and described in appendices attached to the priority filings, which is incorporated herein in this written description. Further, this application incorporates by reference the contents of PCT Appl. No. PCT/IB2010/002054 of the same applicant, entitled FLUID INDICATOR, filed on the 20^{th} of August, 2010.

It should be appreciated that the particular implementations shown and described herein are only representative of the invention and its best mode. Furthermore, any connecting lines shown in the various figures contained herein are intended to represent exemplary functional relationships and/or physical couplings between various elements. It should be noted that many alternative or additional physical connections or functional relationships may be present and apparent to someone of ordinary skill in the field.

Moreover, the apparatus, system and/or method contemplates the use, sale and/or distribution of any goods, services or information having similar functionality described herein.

The the scope of the invention should be determined by the claims appended hereto and their legal equivalents rather than by merely the examples described above. For instance, steps recited in any method or process claims should be construed as being executable in any order and are not limited to the specific order presented in any claim. Further, the elements and/or components recited in any apparatus claims may be assembled or otherwise operationally configured in a variety of permutations to produce substantially the same result as the present invention.

Benefits, other advantages and solutions mentioned herein are not to be construed as necessary, critical, or essential features or components of any or all the claims.

As used herein, the terms "comprises", "comprising", or any variation thereof, are intended to refer to a non-exclusive listing of elements, such that any process, method, article, composition or apparatus of the invention that comprises a list of elements does not include only those elements recited, but may also include other elements described in this specification. The use of the term "consisting" or "consisting of" or "consisting essentially of' is not intended to limit the scope of the invention to the enumerated elements named thereafter, unless otherwise indicated. Other combinations and/or modifications of the above-described elements, materials or structures used in the practice of the present invention may be varied or otherwise adapted by the skilled artisan to other design without departing from the general principles of the invention.

## Claims

1. A visual indicator device comprising:
a bracelet adapted to be worn around an appendage of a wearer;
a transparent capillary chamber (36, 36', 36"), for containing at least one fluid, the capillary chamber matched to an indicia for measuring such fluid, the chamber having a primary length and a width less than the primary length; and
a displacement member functionally disposed at an end of the capillary chamber wherein the displacement member (90) is a piston (35, 166) located at the end of the chamber, and wherein the bracelet is the transparent capillary chamber and the capillary chamber is flexible, **characterized in that** the indicia is disposed along more than half of the entire length of the transparent capillary chamber and **in that** the piston has a rear face and a front driving face to advance the at least one fluid (28) in the capillary chamber (120) along its length, and further **in that** the at least one fluid is in contact with the front face of the piston, the at least one fluid being located between the front face of the piston and the end of the capillary chamber, which is responsive to the piston to move at least one fluid along the capillary chamber.

2. The visual indicator device (10, 10', 10") of claim 1 wherein the primary length is a circumferential length.

3. The visual indicator device (10, 10', 10") of claim 1, wherein the primary length is a spline length.

4. The visual indicator device (10, 10', 10") of claim 1, wherein the at least one fluid (28) is a fluid selected from a group of fluids consisting of a drug, an oil, and a perfume.

5. The visual indicator device (10, 10', 10") of claim 4, wherein the indicator further includes a catheter between the front face of the piston and the end of the capillary chamber connecting to a wearer, and wherein the indicator indicates an amount of such fluid administered to the wearer.

6. The visual indicator device (10, 10', 10") of claim 5, wherein the at least one fluid is dispensed onto an absorptive pad (25) held adjacent to the skin of the wearer.

## Patentansprüche

1. Optische Anzeigervorrichtung, umfassend:
ein Armband, das dazu eingerichtet ist, um an einer Gliedmaße eines Trägers getragen zu werden;
eine transparente Kapillarkammer (36, 36', 36'') zum Enthalten mindestens eines Fluids, wobei die Kapillarkammer auf eine Markierung zum Messen eines solchen Fluids abgestimmt ist, die Kammer weist eine primäre Länge auf und eine Breite, die geringer ist als die primäre Länge; und
ein Verdrängungselement, das funktionell an einem Ende der Kapillarkammer angeordnet ist, wobei das Verdrängungselement (90) ein Kolben (35, 166) ist, der sich an dem Ende der Kammer befindet, und wobei das Armband die transparente Kapillarkammer ist und die Kapillarkammer flexibel ist, **dadurch gekennzeichnet, dass** die Markierung entlang mehr als einer Hälfte der gesamten Länge der transparenten Kapillarkammer angeordnet ist und dass der Kolben eine hintere Seite und eine vordere Antriebsseite aufweist, um das mindestens eine Fluid (28) in der Kapillarkammer (120) entlang ihrer Länge voranzutreiben, und weiter dass das mindestens eine Fluid in Kontakt mit der vorderen Seite des Kolbens ist, wobei das mindestens eine Fluid sich zwischen der vorderen Seite des Kolbens und dem Ende der Kapillarkammer befindet, das auf den Kolben reagiert, um das mindestens eine Fluid entlang der Kapillarkammer zu bewegen.

2. Optische Anzeigervorrichtung (10, 10', 10'') nach Anspruch 1, wobei die primäre Länge eine Umfangslänge ist.

3. Optische Anzeigervorrichtung (10, 10', 10'') nach Anspruch 1, wobei die primäre Länge eine Kurvenlänge ist.

4. Optische Anzeigervorrichtung (10, 10', 10'') nach Anspruch 1, wobei das mindestens eine Fluid (28) ein Fluid ist, das aus einer Gruppe von Fluiden bestehend aus einem Wirkstoff, einem Öl und einem Parfüm ausgewählt ist.

5. Optische Anzeigervorrichtung (10, 10', 10'') nach Anspruch 4, wobei der Anzeiger weiter einen Katheter zwischen der vorderen Seite des Kolbens und dem Ende der Kapillarkammer, das mit einem Träger verbunden ist, umfasst, und wobei der Anzeiger eine Menge eines solchen Fluids anzeigt, die dem Träger verabreicht wird.

6. Optische Anzeigervorrichtung (10, 10', 10'') nach Anspruch 5, wobei das mindestens eine Fluid auf eine absorptionsfähige Kompresse (25) abgegeben wird, die an die Haut des Trägers anliegend gehalten wird.

## Revendications

1. Dispositif d'indicateur visuel comprenant:
un bracelet adapté pour être porté autour d'un membre d'un porteur;
une chambre capillaire transparente (36, 36', 36"), pour contenir au moins un fluide, la chambre capillaire étant mise en correspondance avec un indice pour mesurer ledit fluide, la chambre ayant une longueur primaire et une largeur inférieure à la longueur primaire; et
un élément de déplacement disposé de manière fonctionnelle à une extrémité de la chambre capillaire, dans lequel l'élément de déplacement (90) est un piston (35, 166) situé à l'extrémité de la chambre et dans lequel le bracelet est la chambre capillaire transparente et la chambre capillaire est souple,
**caractérisé en ce que** l'indice est disposé le long de plus de la moitié de la longueur totale de la chambre capillaire transparente et **en ce que** le piston a une face arrière et une face d'entraînement avant pour faire avancer l'au moins un fluide (28) dans la chambre capillaire (120) le long de sa longueur et en outre **en ce que** l'au moins un fluide est en contact avec la face avant du piston, l'au moins un fluide étant situé entre la face avant du piston et l'extrémité de la chambre capillaire qui réagit au piston pour déplacer au moins un fluide le long de la chambre capillaire.

2. Dispositif d'indicateur visuel (10, 10', 10") selon la revendication 1, dans lequel la longueur primaire est une longueur circonférentielle.

3. Dispositif d'indicateur visuel (10, 10', 10") selon la revendication 1, dans lequel la longueur primaire est une longueur cannelée.

4. Dispositif d'indicateur visuel (10, 10', 10") selon la revendication 1, dans lequel l'au moins un fluide (28) est un fluide choisi parmi un groupe de fluides constitué d'un médicament, d'une huile et d'un parfum.

5. Dispositif d'indicateur visuel (10, 10', 10") selon la revendication 4, dans lequel l'indicateur comprend en outre un cathéter entre la face avant du piston et l'extrémité de la chambre capillaire se reliant à un porteur et dans lequel l'indicateur indique une quantité dudit fluide administrée au porteur.

6. Dispositif d'indicateur visuel (10, 10', 10") selon la revendication 5, dans lequel l'au moins un fluide est distribué sur un tampon absorbant (25) maintenu adjacent à la peau du porteur.
